# EUROPEAN PATENT SPECIFICATION

(11) **EP 2 985 025 B1**
(45) Date of publication and mention of the grant of the patent: **17.01.2018**
(21) Application number: 15179438.5
(22) Date of filing: 04.06.2009
(51) Int. Cl.: A61K 31/496, A61K 31/519, A61P 35/00, A61P 3/10, A61P 17/06, A61P 19/02, A61P 25/02, A61P 9/00, A61P 11/06, A61P 27/02, A61P 1/16, A61P 43/00, A61K 9/48

(54) **PHARMACEUTICAL COMBINATION**
PHARMAZEUTISCHE KOMBINATION
ASSOCIATION PHARMACOLOGIQUE

(30) Priority: 06.06.2008 EP 08157749; 08.07.2008 US 78882 P
(43) Date of publication of application: 17.02.2016
(62) Divisional of application: 09757599.7
(73) Proprietor: BOEHRINGER INGELHEIM INTERNATIONAL GMBH, 55216 Ingelheim am Rhein (DE)
(72) Inventor: STEFANIC, Martin Friedrich, 55216 INGELHEIM AM RHEIN (DE); HILBERG, Frank, 55216 INGELHEIM AM RHEIN (DE); KAISER, Rolf, 55216 INGELHEIM AM RHEIN (DE); SHAPIRO, David, WALLINGFORD, CT Connecticut 06492 (US)
(74) Representative: Simon, Elke Anna Maria

(56) References cited:
- EP-A- 0 432 677
- WO-A-2004/017948
- WO-A-2006/067165
- WO-A-2007/057397
- WO-A1-2005/070469
- WO-A1-2006/018182
- WO-A2-01/14379
- WO-A2-2004/096224
- US-A1- 2006 041 013
- Anonymous: "Clinically meaningful data for oral nintedanib in mesothelioma presented at World Conference on Lung Cancer", , 7 December 2016 (2016-12-07), XP055352070, Retrieved from the Internet: URL:https://www.boehringer-ingelheim.com/p ress-release/positive-data-nintedanib-meso thelioma-world-conference-lung-cancer [retrieved on 2017-03-07]

## Description

The present invention relates to a pharmaceutical combination comprising the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone (compound A) or a pharmaceutically acceptable salt thereof and the compound *N*-[4-[2-(2-amino-4,7-dihydro-4-oxo-1*H*-pyrrolo[2,3-*d*]pyrimidin-5-yl)ethyl]benzoyl]-L-Glutamic acid (compound B) or a pharmaceutically acceptable salt thereof for use in a method of treatment of malignant pleural or peritoneal mesothelioma.
Also, the invention relates to the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof for use in a method of treatment of malignant pleural or peritoneal mesothelioma comprising administering to a patient in need thereof an effective amount of said compound before, after or simultaneously with an effective amount of the compound *N*-[4-[2-(2-amino-4,7-dihydro-4-oxo-1*H*-pyrrolo[2,3-*d*]pyrimidin-5-yl)ethyl]benzoyl]-L-Glutamic acid or a pharmaceutically acceptable salt thereof

### Background to the invention

The compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone (compound A) is an innovative compound having valuable pharmacological properties, especially for the treatment of oncological diseases, immunologic diseases or pathological conditions involving an immunologic component, or fibrotic diseases.

The chemical structure of this compound is depicted below as Formula A.

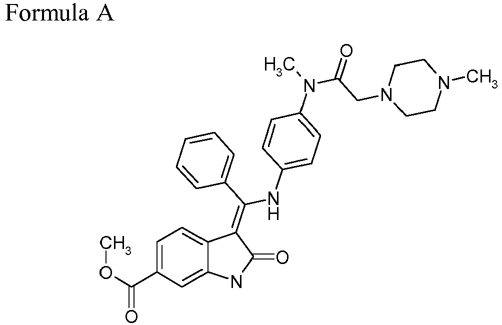

The base form of this compound is described in WO 01/27081, the monoethanesulphonate salt form is described in WO 2004/013099 and various further salt forms are presented in WO 2007/141283. The use of this molecule for the treatment of immunologic diseases or pathological conditions involving an immunologic component is being described in WO 2004/017948 , the use for the treatment of oncological diseases is being described in WO 2004/096224 and the use for the treatment of fibrotic diseases is being described in WO 2006/067165.

The monoethanesulphonate salt form of this compound presents properties which makes this salt form especially suitable for development as medicament. The chemical structure of 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone-monoethanesulphonate is depicted below as Formula A1.

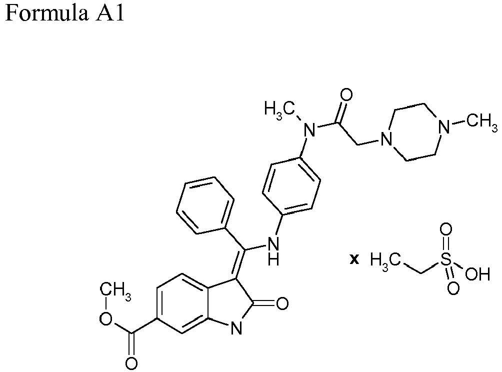

Preclinical studies have shown that this compound is a highly potent, orally bioavailable inhibitor of vascular endothelial growth factor receptors (VEGFRs), platelet-derived growth factor receptors (PDGFRs) and fibroblast growth factor receptors (FGFRs) that suppresses tumor growth through mechanisms inhibiting tumor neovascularization. It has further been shown that this compound inhibits signalling in endothelial- and smooth muscle cells and pericytes, and reduces tumor vessel density.

Furthermore, this compound shows *in vivo* anti-tumor efficacy in all models tested so far at well tolerated doses. The following table shows the results of the *in vivo* anti-tumor efficacy testing in xenograft models and in a syngeneic rat tumor model.

| **Cancer** | **Model** | **Efficacy** |
|---|---|---|
| Colorectal | HT-29 | T/C 16% @ 100mg/kg/d |
| | HT-29 large tumors | tumor volume reduction |
| Glioblastoma | GS-9L syngeneic rat | T/C 32% @ 50mg/kg/d |
| Head and neck | FaDu | T/C 11% @ 100mg/kg/d |
| Lung (non-small-cell) | NCI-H460 | T/C 54% @ 25mg/kg/d |
| | Calu-6 | T/C 24% @ 50mg/kg/d |
| Ovarian | SKOV3 | T/C 19% @ 50mg/kg/d |
| Prostate (hormone-dependent) | PAC-120 | T/C 34% @ 100mg/kg/d |
| Renal | Caki-1 | T/C 13% @ 100mg/kg/d |
| Pancreas (murine transgenic) | Rip-Tag | interference with tumor formation |

| | | |
|---|---|---|
| T/C represents the reduction of tumor size in % of the control | | |

This compound is thus suitable for the treatment of diseases in which angiogenesis or the proliferation of cells is involved.

The compound *N*-[4-[2-(2-amino-4,7-dihydro-4-oxo-1*H*-pyrrolo[2,3-*d*]pyrimidin-5-yl)ethyl]benzoyl]- L-Glutamic acid (compound B) is an antifolate that inhibits *de novo* DNA synthesis pathways and has demonstrated clinical benefit in patients with advanced malignant pleural mesothelioma (in combination with cisplatin) whose disease is unresectable or who are not eligible for curative treatment. This compound has also shown a similar efficacy compared to docetaxel in patients suffering from advanced or metastatic non small cell lung cancer (NSCLC) that failed one prior first line chemotherapy. The pyrrolopyrimidine-based nucleus of the compound exerts its antineoplastic activity by disrupting folate-dependent metabolic processes essential for cell replication. In vitro data have shown that this molecule inhibits the thymidylate synthase (TS), the dihydrofolate reductase (DHFR), and the glycinamide ribonucleotide formyltransferase (GARFT). All these enzymes are folate-dependent enzymes which are involved in the de novo biosynthesis of thymidine and purine nucleotides.

The structure of the compound *N*-[4-[2-(2-amino-4,7-dihydro-4-oxo-1*H*-pyrrolo[2,3-*d*]pyrimidin-5-yl)ethyl]benzoyl]-L-Glutamic is depicted below as Formula B. This compound is described for example in EP 00432677, and further known as pemetrexed.

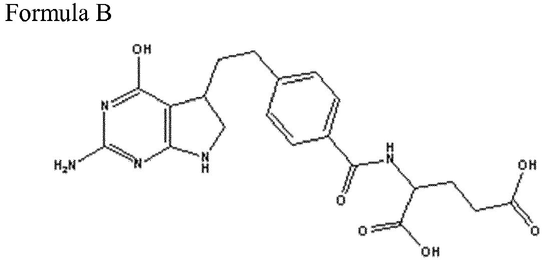

Pemetrexed is approved since 2004 in the USA in its disodium salt form for use in combination with cisplatin for the treatment of patients with malignant pleural mesothelioma and since 2005 for the treatment of second line NSCLC patients. It is commercialized under the trade name Alimta®.

The approved active ingredient pemetrexed disodium heptahydrate has the chemical name *N*-[4-[2-(2-amino-4,7-dihydro-4-oxo-1*H*-pyrrolo[2,3-*d*]pyrimidin-5-yl)ethyl]benzoyl]-L-Glutamic acid, disodium salt, heptahydrate and is depicted below as Formula B1. It is a white to almost-white solid with a molecular formula of C₂₀H₁₉N₅Na₂O₆•7H₂O and a molecular weight of 597.49.

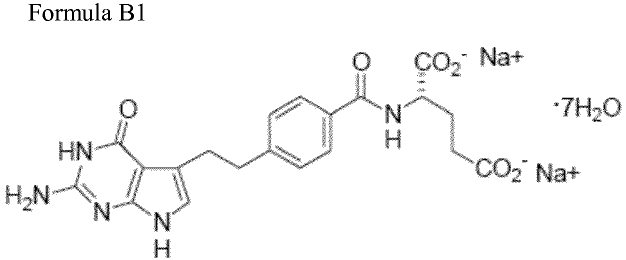

Alimta® is supplied as a sterile lyophilized powder for intravenous infusion available in single-dose vials. The product is a white to either light yellow or green-yellow lyophilized solid. Each 500-mg vial of Alimta® contains pemetrexed disodium equivalent to 500 mg pemetrexed and 500 mg of mannitol. Hydrochloric acid and/or sodium hydroxide may have been added to adjust the pH.

The aim of the present disclosure is to provide a pharmaceutical combination for the treatment of diseases which involve cell proliferation, or involve migration or apoptosis of myeloma cells, or angiogenesis on the basis of the above mentioned compounds. Such specific pharmaceutical combination is not known from the prior art. Its advantages are the potential for an improved clinical benefit for cancer patients treated with this pharmaceutical combination facilitated by one or more of the following mechanisms:
- Additive or synergistic antitumor effect through the combination of two different anticancer principles and target structures;
- Additive or synergistic antitumor effect through an increased availability of compound B1 in cancer lesions by lowering of the intratumoural pressure with compound A1;
- Prevention of the pro-angiogenic rebound after chemotherapeutic intervention with compound B1 with or without radiotherapy;
- Maintenance of the tumour response or of the tumour stabilisation achieved with the combination of both compounds A1 and B1, or with compound A1 alone after combination of compound A1 and B1, or with compound B1 alone by subsequent treatment with compound A1. A treatment effect of compound A1 may prevail even after toxicity-guided dose reductions from the maximum tolerated dose in single patients.

The method of use of pemetrexed for the manufacture of a medicament for the treatment of mesothelioma is described in WO 01/14379.
WO 2004/096224 discloses a pharmaceutical combination comprising nintedanib monoethanesulfonate and further therapeutic agents for use in a method of treatment of malignant mesotheliomas.
WO 2005/070469 claims a method of treating malignant mesothelioma in an animal with a combination of an antisense oligonucleotide and pemetrexed.
The combination of a Polo-like kinase inhibitor with nintedanib monoethanesulfonate or with pemetrexed for the treatment of malignant mesotheliomas is described in WO 2006/018182.
The combination of L-alanosine with pemetrexed was tested in tumor cell lines from mesothelioma (US 2006/0041013).

### Summary of the invention

A first object of the present invention is a pharmaceutical combination comprising the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, preferably the monoethanesulphonate salt form, and the compound *N*-[4-[2-(2-amino-4,7-dihydro-4-oxo-1*H*-pyrrolo[2,3-*d*]pyrimidin-5-yl)ethyl]benzoyl]- L-Glutamic acid or a pharmaceutically acceptable salt thereof, preferably the disodium salt form for use in a method of treatment of malignant pleural or peritoneal mesothelioma.

A further object of the present invention is the above pharmaceutical combination, which is further in the form of a combined preparation for simultaneous, separate or sequential use.

A further object of the present invention is the above pharmaceutical combination for the above-mentioned use, which is further adapted for a co-treatment with radiotherapy.

A further object of the present invention is the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, preferably the monoethanesulphonate salt form, for use in the treatment of malignant pleural or peritoneal mesothelioma comprising administering to a patient in need thereof an effective amount of said compound before, after or simultaneously with an effective amount of the compound *N*-[4-[2-(2-amino-4,7-dihydro-4-oxo-1*H*-pyrrolo[2,3-*d*]pyrimidin-5-yl)ethyl]benzoyl]-L-Glutamic acid or a pharmaceutically acceptable salt thereof, preferably the disodium salt form.

### Legend to the Figures

Figure 1 : Tumor volume evolution over time of Calu-6 NSCLC Xenografts without treatment (T/C value of the control treated group equals 100% at the end of the experiment) after treatment with compound A1 (T/C value 33%), after treatment with compound B1 (T/C value 46%) and after treatment with a combination of compound A1 and compound B1 (T/C value 15%).
Figure 2 : % of change of body weight of the animals during the treatment as shown in Figure 1.

### Detailed description of the invention

As already mentioned hereinbefore, the present disclosure relates to a pharmaceutical combination comprising an effective amount of the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof and an effective amount of the compound *N*-[4-[2-(2-amino-4,7-dihydro-4-oxo-1*H-*pyrrolo[2,3-*d*]pyrimidin-5-yl)ethyl]benzoyl]-L-Glutamic acid or a pharmaceutically acceptable salt thereof

A combination treatment of the present disclosure as defined herein may be achieved by way of the simultaneous, sequential or separate administration of the individual components of said treatment. A combination treatment as defined herein may be applied as a sole therapy or may involve surgery or radiotherapy or an additional chemotherapeutic or targeted agent in addition to a combination treatment of the disclosure. Surgery may comprise the step of partial or complete tumour resection, prior to, during or after the administration of the combination treatment as described herein.

According to another aspect of the present disclosure, the effect of a method of treatment of the present disclosure is expected to be at least equivalent to the addition of the effects of each of the components of said treatment used alone, that is, of each of the compounds and ionising radiation used alone.

According to another aspect of the present disclosure the effect of a method of treatment of the present disclosure is expected to be greater than the addition of the effects of each of the components of said treatment used alone, that is, of each of the compounds and ionising radiation used alone.

According to another aspect of the present disclosure the effect of a method of treatment of the present disclosure is expected to be a synergistic effect. A combination treatment is defined as affording a synergistic effect if the effect is therapeutically superior, as measured by, for example, the extent of the response, the duration of response, the response rate, the stabilisation rate, the duration of stabilisation, the time to disease progression, the progression free survival or the overall survival, to that achievable on dosing one or other of the components of the combination treatment at its conventional dose. For example, the effect of the combination treatment is synergistic if the effect is therapeutically superior to the effect achievable with one component alone. Further, the effect of the combination treatment is synergistic if a beneficial effect is obtained in a group of patients that does not respond (or responds poorly) to one component alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the duration of response, the response rate, the stabilisation rate, the duration of stabilisation, the time to disease progression, the progression free survival or the overall survival, is equivalent to that achievable on dosing conventional amounts of the components of the combination treatment.

In particular, synergy is deemed to be present if the conventional dose of one of the components may be reduced without detriment to one or more of the extent of the response, the duration of response, the response rate, the stabilisation rate, the duration of stabilisation, the time to disease progression, the progression free survival or the overall survival" in particular without detriment to the duration of the response, but with fewer and/or less troublesome side-effects than those that occur when conventional doses of each component are used.

As stated above the combination treatments of the present disclosure as defined herein are of interest for their antiangiogenic and/or vascular permeability effects. Angiogenesis and/or an increase in vascular permeability is present in a wide range of disease states including cancer (including Kaposi's sarcoma, leukaemia, multiple myeloma and lymphoma), diabetes, psoriasis, rheumatoid arthritis, haemangioma, acute and chronic nephropathies, atheroma, arterial restenosis, autoimmune diseases, acute inflammation, asthma, lymphoedema, endometriosis, dysfunctional uterine bleeding, fibrosis, cirrhosis and ocular diseases with retinal vessel proliferation including age-related macular degeneration. Combination treatments of the present disclosure are expected to be particularly useful in the prophylaxis and treatment of diseases such as cancer and Kaposi's sarcoma. In particular such combination treatments are expected to slow advantageously the growth of primary and recurrent solid tumours of, for example, the colon, pancreas, brain, bladder, ovary, breast, prostate, lungs and skin. Combination treatments of the present disclosure are expected to slow advantageously the growth of tumours in lung cancer, including malignant pleural mesothelioma, small cell lung cancer (SCLC) and non- small cell lung cancer (NSCLC), head and neck cancer, oesophageal cancer, stomach cancer, colorectal cancer, gastrointestinal stromal tumor (GIST), pancreatic cancer, hepatocellular cancer, breast cancer, renal cell cancer and urinary tract cancer, prostate cancer, ovarian cancer, brain tumors, sarcomas, skin cancers, and hematologic neoplasias (leukemias, myelodyplasia, myeloma, lymphomas).

More particularly such combination treatments of the disclosure are expected to inhibit any form of cancer associated with VEGF including leukaemia, multiple myeloma and lymphoma and also, for example, to inhibit the growth of those primary and recurrent solid tumors which are associated with VEGF, especially those tumors which are significantly dependent on VEGF for their growth and spread, including for example, certain tumours of the colon (including rectum), pancreas, brain, kidney, hepatocellular cancer, bladder, ovary, breast, prostate, lung, vulva, skin and particularly malignant pleural mesothelioma and NSCLC. More especially combination treatments of the present invention are expected to slow advantageously the growth of tumours in malignant pleural mesothelioma. More especially combination treatments of the present disclosure are expected to slow advantageously the growth of tumors in non-small cell lung cancer (NSCLC).

In another aspect of the present disclosure , the combination is expected to inhibit the growth of those primary and recurrent solid tumors which are associated with VEGF, especially those tumors which are significantly dependent on VEGF for their growth and spread.

The advantages of the present invention are the potential for an improved clinical benefit for cancer patients treated with this pharmaceutical combination involving one or more of the following mechanisms:
- Additive or synergistic antitumor effect mediated by the combination of two different anticancer principles and target structures: Compound A1 is an antiangiogenic compound targeting the tumor vasculature (endothelial cells, pericytes, and smooth muscle cells) with suppression of tumor (re-)growth and metastatic spread; compound B1 is a cyctotoxic agent interacting with *de novo* DNA synthesis pathways. Unlike normal cells, cancer cells are genetically instable, causing them to replicate inaccurately. As tumors progress, this genetic instability leads to subpopulations of tumor cells with different biological features. An antitumor treatment like compound B1 may terminate even the majority of tumor tissue, however, finally, some cell clones will become refractory. After the treatment-sensitive cells have been killed, the resistant cells may rapidly divide again to restore a tumor that is inherently resistant to the therapy. Therefore, simultaneous targeting of different principles driving cancer growth and spread with the described combination of compound A1 and compound B1 reduces the risk of primary and secondary tumor resistance and tumor escape as well. The validity of such approaches has been demonstrated for combination and multimodality treatment in a variety of solid and hematologic human malignancies, but not for the combination object of the present invention, i.e. the combination of compound A1 and compound B1. Of importance in the context of the present invention may be the fact that compound A1 primarily acts on the genetically stable cells of the tumor vasculature which are less prone to spontaneous mutation and resistance development as compared to the malignant cells.
- Additive or synergistic antitumor effect through an increased availability of compound B1 in cancer lesions by lowering of the intratumoural pressure with compound A1. Treatment with compound A1 may significantly reduce vessel density and permeability thereby contributing to an increase in net tumor perfusion and a reduction of the intratumoral pressure. This process may lead to an increased availability of molecules like compound B1 within the tumor lesions.
- Prevention of the pro-angiogenic rebound by compound A1 after chemotherapeutic intervention with compound B1 with or without radiotherapy. Conventional chemotherapy with compound B1 or with radiotherapy may be followed by a so-called proangiogenic rebound of soluble pro-angiogenic factors and bone marrow derived circulating endothelial cells which may diminish the therapeutic effect and help the tumor to compensate the damage caused by compound B1 or radiotherapy. Eliminating this effect during the compound B1-free or radiotherapy-free break periods by continued treatment with compound A1 may compromise this robust repair process and lead to an increased and more sustainable antitumor effect.
- Maintenance of the tumour response or of the tumour stabilisation achieved with the combination of both compounds A1 and B1, or with compound A1 alone after combination of compound A1 and B1, or with compound B1 alone by subsequent treatment with compound A1.
- Despite its proven merits, treatment with conventional chemotherapeutics like with compound B1 is limited mainly by its unevitable toxicities on dividing healthy tissues and the often relatively rapid emergence of tumor resistance and subsequent tumor relapse or progression. Therefore, an approach to maintain the benefits achieved with chemotherapy, here with compound B1, is of high importance and value to the cancer patient. Treatment with compound A1 as an add-on to treatment with compound B1 and also after completion of the treatment with compound B1 has the potential to achieve this goal, as may be assessed by a prolongation of the duration of tumour response or of the tumor stabilisation, progression free survival, and overall survival. The following clinical Phase II data on maintenance treatment with compound A1 alone that were collected in patients with relapsed ovarian cancer after completion of chemotherapy further support the concept of maintenance treatment.

### Pre clinical study results

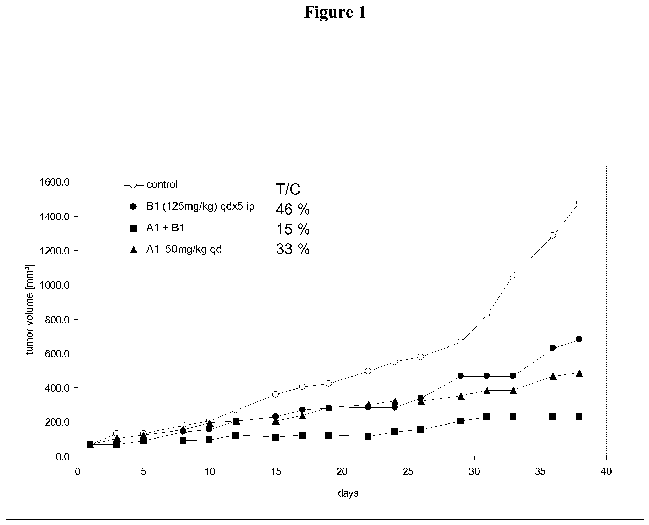

In order to analyse the anti-tumor effects of combining the inhibition of tumor angiogenesis by interfering with the VEGFR signaling cascade with the established anti-proliferative treatment modality of NSCLC with compound B1, the following in vivo experiment was performed. Nude mice carrying established subcutaneous Calu-6 xenografts (human NSCLC tumor cell line) were randomized and treated with either compound B1 or compound A1 alone or with the combination of both drugs. After 38 days of treatment the tumors on the control treated mice had reached the endpoint and were in average ∼1400 mm³ in volume. The results of Figure 1 show that the combination of suboptimal doses of compound A1 and compound B1 results in improved antitumour efficacy with a T/C value of 15 % compared to single agent treatments (T/C values of 33% and 46 %, respectively).

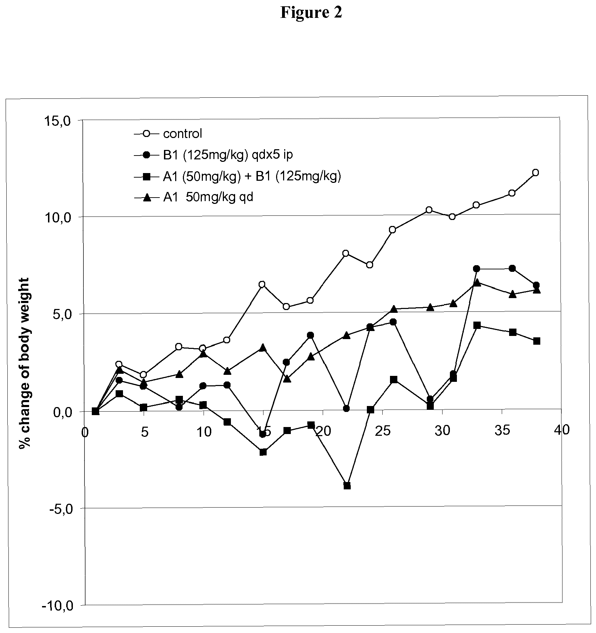

The results of Figure 2 show that the doses applied during this tumor experiment did not lead to weight loss in the treated mice. The weight gain of the mice in the treatment groups in comparison to the weight of the control mice was reduced, but nevertheless well tolerated.

### Phase I study results

A further study was performed, namely a Phase I, open-label dose escalation study to investigate the combination of compound A1 together with a standard dose of compound B1 in previously treated patients with recurrent advanced stage NSCLC. The potential additive or synergistic effects of novel therapeutic regimens may make combinations of these agents particularly attractive for the treatment of patients with advanced NSCLC compared to a single agent alone.
The primary objectives of this trial were to determine the safety, tolerability, Maximum Tolerated Dose (MTD) and pharmacokinetics of compound Alin combination with a standard dose of compound B1.

### Methods

Patients with advanced stage NSCLC, PS 0-1, previously treated with one first line platinum-based chemotherapy regimen were eligible for this trial. The trial was an open label, dose escalation design with compound A1 at a starting dose of 100 mg bid, taken on days 2-21, combined with standard dose compound B1 (500 mg/m²) given as a 10 minute intravenous infusion on day 1 of a 21 day cycle. Patients could be treated for a minimum of four and a maximum of six cycles of the combination therapy, with an option of compound A1 monotherapy following the completion of the combination stage. Compound A1 was escalated at doses of 50 mg per cohort until the MTD dose was determined. The MTD was defined as the dose of compound A1 which was one dose cohort below the dose at which two or more out of six patients experienced dose limiting toxicity (DLT) in the first treatment cycle. Tumor assessments were performed at screening and after every second treatment cycle according to RECIST (Response Evaluation Criteria in Solid Tumors).

### Results

Twenty-six patients (13 male, 13 female, median age of 61.5 years) in total and 12 at the MTD were treated in this study. The MTD dose of compound A1 was determined to be 200 mg *bid* (twice a day) in combination with a standard dose of compound B1. Generally the combination of compound A1 and compound B1 was well tolerated. During the first treatment course, 7 patients developed a Dose Limiting Toxicity (DLT): 1 out of 6 patients at 100 mg compound A1 *bid,* 1 out of 6 patients at 150 mg compound A1 *bid,* 3 out of 12 patients at 200 mg compound A1 *bid,* and 2 out of 2 patients at the 250 mg compound A1 *bid.* These DLTs included elevated liver enzymes, gastrointestinal events including vomiting and nausea, fatigue and confusion and were all of CTC (Common Toxicity Criteria of the National Institute of Health) Grade 3. These events resolved following discontinuation of the study medication. No CTC Grade 4 events occurred in the study. Best responses by RECIST included (20 evaluable for response) 1 Complete Response (CR) and 13 patients with Stable Disease (SD). The patient with the CR has been maintained on compound A1 monotherapy for a period of over 63 weeks. Half of the 26 treated patients had Stable Disease (SD) as the best overall response according to the investigators' assessments, with the Maximum Tolerated Dose (MTD) group having 58.3% SD as the best overall response. Median Progression Free Survival (PFS) for all patients was 5.4 months.

### Conclusions

The combination of compound A1 and compound B1 in previously treated NSCLC patients was shown to be safe and well tolerated in this study. The Maximum Tolerated Dose (MTD) dose of compound A1 was 200 mg *bid* (twice a day) when given with compound B1 at a dose of 500 mg/m² (recommended dose of pemetrexed for NSCLC treatment). Signs of clinical efficacy were observed in the small number of patients treated in this trial. One patient is on complete response since three years.

### Phase II study results

### Phase II trial in patients with advanced non-small cell lung cancer

This study was conducted as a Phase II double-blind, randomized study of two different doses of orally administered compound A1 in patients with advanced non-small-cell lung cancer who had failed at least one prior chemotherapy regimen. The primary efficacy endpoints evaluated were response rate and time to progression. Important secondary endpoints were survival and tolerability of compound A1.

### Methods

Patients were randomly assigned to receive compound A1 at a dose of 250 mg twice daily or 150 mg twice daily. The dose of compound A1 could be reduced stepwise to no lower than 100 mg twice daily in case of undue toxicity that would prevent chronic treatment. Patients were treated until diagnosis of progression of the underlying lung cancer disease. Progressive disease, for the analysis of the primary endpoint, was defined as radiological evidence of tumour progression according to RECIST criteria.

### Results

This randomized study enrolled 73 patients in total, 36 patients at the dose of 250 mg twice daily and 37 patients at the dose of 150 mg twice daily.
The ECOG performance status score is a scale from 0 to 5 with criteria used by doctors and researchers to assess how a patient's disease is progressing, assess how the disease affects the daily living abilities of the patient, and determine appropriate treatment and prognosis (Oken, M.M., Creech, R.H., Tormey, D.C., Horton, J., Davis, T.E., McFadden, E.T., Carbone, P.P.: Toxicity And Response Criteria Of The Eastern Cooperative Oncology Group. Am J Clin Oncol 5:649-655, 1982). Progression Free Survival (PFS) time is defined as the length of time during and after treatment in which a patient is living with a disease that does not get worse. Overall Survival (OS) time is defined as the length of time a patient lives after he is diagnosed with or treated for a disease.
Compound A1 at 150 mg twice daily and 250 mg twice daily were equivalent in terms of median Progression Free Survival (PFS) time (48 vs. 53 days). The corresponding Overall Survival (OS) times were 144 days for patients receiving the 150 mg dose and 208 days for patients receiving the 250 mg dose. When considering patients with a baseline ECOG of 0 or 1, the median PFS was greater compared with all patients; as for all patients, median PFS was independent of dose (150 mg twice daily: 81 days; 250 mg twice daily: 85 days). In the subgroup with ECOG 0 or 1, clinical benefit was achieved by nearly 60% of patients; one of the 17 patients with baseline ECOG of 2 had stable disease. One patient treated with 250 mg of compound A1 twice daily sustained a 74% reduction (partial response) in tumor size through 9 months. The median overall survival (OS) of all patients was 153 days. (ECOG 0-2) and patients with ECOG score of 0-1 had a median OS of 264 days.

### Conclusion

Compound A1 showed encouraging signs of efficacy in non-small cell lung cancer patients with ECOG performance score 0 to 1. There was no evidence of a difference in efficacy between the two dosages of compound A1.

### Phase II maintenance trial in patients with advanced ovarian cancer

A double-blind, randomized Phase II trial was performed to assess efficacy and safety of compound A1 as maintenance therapy in a population of patients who had experienced an early (< 12 months after preceding chemotherapy, indicating a relative refractoriness to platinum based standard therapy) relapse of ovarian cancer. Therapy with compound A1 was to start as maintenance after achievement of a clinical benefit to the cytotoxic induction treatment of the relapse. The aim of the trial was to explore the therapeutic potential of compound A1 as compared to placebo, i.e. whether compound A1 showed signs of sustainment of the clinical benefit (objective response or tumour stabilization) to relapse therapy induced by an immediately preceding cytotoxic regimen. The primary efficacy endpoint of this trial was the Progression Free Survival Rate (PFSR) at 9 months after start of treatment with compound A1. As secondary endpoints PFS rate at 3 months and 6 months, respectively, and time to next anti-tumour treatment were evaluated.

### Methods

Patients were randomly assigned to receive compound A1 at a dose of 250 mg twice daily or matching placebo. The dose of compound A1 or matching placebo could be reduced stepwise to no lower than 100 mg twice daily in case of undue toxicity that would prevent chronic treatment. Patients were treated until diagnosis of progression of the underlying ovarian cancer disease. Progressive disease, for the analysis of the primary endpoint, was defined as either radiological progression, or tumour marker (CA-125) progression.

### Results

In total, 84 patients were entered into the trial. 44 patients were randomised to receive compound A1 at a dose of 250 mg twice daily, and 40 patients to receive matching placebo. One patient had to be excluded from the analysis in the compound A1 arm. Overall, patient characteristics were well balanced between treatment arms, if at all there was a bias towards patients with worse prognosis in the compound A1 arm (more patients with metastases, especially with liver metastases, higher mean baseline CA-125, higher percentage of patients with later lines of therapy [2 or more previous therapies]).
According to the preliminary data output from 19 Nov 2008, the PFS rate at 9 months (36 weeks) was 16.5% in the compound A1 arm, and 6.4% in the placebo arm. The PFS rate at 6 months (24 weeks) was 28.3% in the compound A1 arm, and 19.2% in the placebo arm. The PFS rate was not different between arms at 3 months (12 weeks; the first time point of routine imaging). Overall, the likelihood to remain free of progression was higher for patients treated with compound A1. All five patients who remained on treatment until completion of the 9 months study period were treated in the compound A1 arm.
Progressive disease could be diagnosed due to a rise of the tumour marker only ("tumour marker progression"). Based on radiological data, disregarding tumour marker progression, median time to progression was 143 days (95% CI 82-175 days) for patients treated with compound A1, and 85 days (95% CI 78-89 days) for placebo. The time between tumour marker progression and radiological progression also was longer in the compound A1 arm.

### Conclusion

The analysis of the trial suggests that compound A1 given as a long-term treatment may be active in maintaining the clinical benefit achieved with chemotherapy by delaying the further progression of the tumour disease under treatment. Toxicity guided dose reductions to no lower than 100 mg twice daily are appropriate.

### Further embodiments

Further pharmaceutically acceptable salts of the compounds of the combination in accordance with the present disclosure than those already described hereinbefore may, for example, include acid addition salts. Such acid addition salts include, for example, salts with inorganic or organic acids affording pharmaceutically acceptable anions such as with hydrogen halides or with sulphuric or phosphoric acid, or with trifluoroacetic, citric or maleic acid. In addition, pharmaceutically acceptable salts may be formed with an inorganic or organic base which affords a pharmaceutically acceptable cation. Such salts with inorganic or organic bases include for example an alkali metal salt, such as a sodium or potassium salt and an alkaline earth metal salt such as a calcium or magnesium salt.

In accordance with the present disclosure, the compounds of the combination may be formulated using one or more pharmaceutically acceptable excipients or carriers, as suitable. Suitable formulations for both compounds A1 and B1 have already been described in the literature and in patent applications related to these compounds. These formulations are incorporated herein by reference.

The formulation for the compound of formula A1 may be a lipid suspension of the active substance comprising preferably a lipid carrier, a thickener and a glidant/solubilizing agent, most preferably in which the lipid carrier is selected from corn oil glycerides, diethylenglycolmonoethylether, ethanol, glycerol, glycofurol, macrogolglycerolcaprylocaprate, macrogolglycerollinoleate, medium chain partial glycerides, medium chain triglycerides, polyethylene glycol 300, polyethylene glycol 400, polyethylene glycol 600, polyoxyl castor oil, polyoxyl hydrogenated castor oil, propylene glycol monocaprylate, propylene glycol monolaurate, refined soybean oil, triacetin, triethyl citrate, or mixtures thereof, the thickener is selected from oleogel forming excipients, such as Colloidal Silica or Bentonit, or lipophilic or amphiphilic excipients of high viscosity, such as polyoxyl hydrogenated castor oil, hydrogenated vegetable oil macrogolglycerol-hydroxystearates, macrogolglycerol-ricinoleate or hard fats, and the glidant/solubilizing agent is selected from lecithin, optionally further comprising one or more macrogolglycerols, preferably selected from macrogolglycerol-hydroxystearate or macrogolglycerol-ricinoleate. The lipid suspension formulation may be prepared by conventional methods of producing formulations known from the literature, i.e. by mixing the ingredients at a pre-determined temperature in a pre-determined order in order to obtain a homogenized suspension.

The above formulation may be preferably incorporated in a pharmaceutical capsule, preferably a soft gelatin capsule, characterised in that the capsule shell comprises e.g. glycerol as plasticizing agent, or a hard gelatin or hydroxypropylmethylcellulose (HPMC) capsule, optionally with a sealing or banding. The capsule pharmaceutical dosage form may be prepared by conventional methods of producing capsules known from the literature. The soft gelatin capsule may be prepared by conventional methods of producing soft gelatin capsules known from the literature, such as for example the "rotary die procedure", described for example in Swarbrick, Boylann, Encyclopedia of pharmaceutical technology, Marcel Dekker, 1990, Vol. 2, pp 269 ff or in Lachmann et al., "The Theory and Practice of Industrial Pharmacy", 2nd Edition, pages 404-419, 1976, or other procedures, such as those described for example in Jimerson R. F. et al., "Soft gelatin capsule update", Drug Dev. Ind. Pharm., Vol. 12, No. 8-9, pp. 1133-44, 1986.

The above defined formulation or the above defined capsule may be used in a dosage range of from 0.1 mg to 20 mg of active substance/ kg body weight, preferably 0.5 mg to 4 mg active substance /kg body weight.

The above defined capsules may be packaged in a suitable glass container or flexible plastic container, or in an aluminium pouch or double poly bag.

The following examples of carrier systems (formulations), soft gelatin capsules, bulk packaging materials, and of a manufacturing process are illustrative of the present invention and shall in no way be construed as a limitation of its scope.

### Examples of carrier systems (formulations), soft gelatin capsules, bulk packaging materials, and of a manufacturing process for the preparation of a lipid suspension formulation of compound A1

The active substance in all the Examples 1 to 10 is 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate (compound A1).

### Example 1

Lipid based carrier system

| **Formulation** | **A** | **B** | **C** |
|---|---|---|---|
| **Ingredients** | **[%]** | **[%]** | **[%]** |
| Active Substance | 43.48 | 43.48 | 43.48 |
| Triglycerides, Medium-Chain | 28.70 | 37.83 | 38.045 |
| Hard fat | 27.39 | 18.26 | 18.26 |
| Lecithin | 0.43 | 0.43 | 0.215 |
| Total (Fillmix) | 100.00 | 100.00 | 100.00 |

### Example 2

Lipid based carrier system with additional surfactant

| **Ingredients** | **[%]** |
|---|---|
| Active Substance | 42.19 |
| Triglycerides, Medium-Chain | 41.77 |
| Hard fat | 12.66 |
| Cremophor RH40 | 2.95 |
| Lecithin | 0.42 |
| Total (Fillmix) | 100.00 |

### Example 3

Hydrophilic carrier system

| **Ingredients** | **[%]** |
|---|---|
| Active Substance | 31.75 |
| Glycerol 85% | 3.17 |
| Purified Water | 4.76 |
| Macrogol 600 | 58.10 |
| Macrogol 4000 | 2.22 |
| Total (Fillmix) | 100.00 |

### Example 4

Soft gelatin capsule containing 50 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance | Active Ingredient | 60.20 | 60.20 | 60.20 |
| Triglycerides, Medium-chain | Carrier | 40.95 | 53.70 | 54.00 |
| Hard fat | Thickener | 38.25 | 25.50 | 25.50 |
| Lecithin | Wetting agent / Glidant | 0.60 | 0.60 | 0.30 |
| Gelatin | Film-former | 72.25 | 72.25 | 72.25 |
| Glycerol 85% | Plasticizer | 32.24 | 32.24 | 32.24 |
| Titanium dioxide | Colorant | 0.20 | 0.20 | 0.20 |
| Iron oxide A | Colorant | 0.32 | 0.32 | 0.32 |
| Iron oxide B | Colorant | 0.32 | 0.32 | 0.32 |
| **Total Capsule Weight** | | **245.33** | **245.33** | 245.33 |

### Example 5

Soft gelatin capsule containing 100 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance | Active Ingredient | 120.40 | 120.40 | 120.40 |
| Triglycerides, Medium-chain | Carrier | 81.90 | 107.40 | 106.8 |
| Hard fat | Thickener | 76.50 | 51.00 | 51.00 |
| Lecithin | Wetting agent / Glidant | 1.20 | 1.20 | 1.80 |
| Gelatin | Film-former | 111.58 | 111.58 | 111.58 |
| Glycerol 85% | Plasticizer | 48.79 | 48.79 | 48.79 |
| Titanium dioxide | Colorant | 0.36 | 0.36 | 0.36 |
| Iron oxide A | Colorant | 0.06 | 0.06 | 0.06 |
| Iron oxide B | Colorant | 0.17 | 0.17 | 0.17 |
| **Total Capsule Weight** | | **440.96** | **440.96** | **440.96** |

### Example 6

Soft gelatin capsule containing 125 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance | Active Ingredient | 150.50 | 150.50 | 150.50 |
| Triglycerides, Medium-chain | Carrier | 102.375 | 134.25 | 133.5 |
| Hard fat | Thickener | 95.625 | 63.75 | 63.75 |
| Lecithin | Wetting agent / Glidant | 1.50 | 1.50 | 2.25 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **556.04** | **556.04** | **556.04** |

### Example 7

Soft gelatin capsule containing 150 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance | Active Ingredient | 180.60 | 180.60 | 180.60 |
| Triglycerides, Medium-chain | Carrier | 122.85 | 161.10 | 160.20 |
| Hard fat | Thickener | 114.75 | 76.50 | 76.50 |
| Lecithin | Wetting agent / Glidant | 1.80 | 1.80 | 2.70 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **626.04** | **626.04** | **626.04** |

### Example 8

Soft gelatin capsule containing 200 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance | Active Ingredient | 240.80 | 240.80 | 240.80 |
| Triglycerides, Medium-chain | Carrier | 163.30 | 214.80 | 216.00 |
| Hard fat | Thickener | 153.50 | 102.00 | 102.00 |
| Lecithin | Wetting agent / Glidant | 2.40 | 2.40 | 1.20 |
| Gelatin | Film-former | 203.19 | 203.19 | 203.19 |
| Glycerol 85% | Plasticizer | 102.61 | 102.61 | 102.61 |
| Titanium dioxide | Colorant | 0.57 | 0.57 | 0.57 |
| Iron oxide A | Colorant | 0.90 | 0.90 | 0.90 |
| Iron oxide B | Colorant | 0.90 | 0.90 | 0.90 |
| **Total Capsule Weight** | | **868.17** | **868.17** | **868.17** |

### Example 9

Bulk packaging materials for the packaging of the soft gelatin capsules of above examples 1 to 4 may be aluminium pouches or double poly bags.

### Example 10

In the following, a manufacturing process for the preparation of a lipid suspension formulation of the active substance and a process for the encapsulation are described.
a: Hard fat and parts of Medium-chain triglycerides are pre-mixed in the processing unit. Subsequently lecithin, the rest of medium-chain triglycerides and the active substance are added. The suspension is mixed, homogenized, deaerated and finally sieved to produce the formulation (Fillmix).
b. The gelatin basic mass components are mixed and dissolved at elevated temperature. Then, the corresponding colours and additional water are added and mixed, producing the Coloured Gelatin Mass.
c. After adjustment of the encapsulation machine, Fillmix and Coloured Gelatin Mass are processed into soft gelatin capsules using the rotary-die process. This process is e.g. described in Swarbrick, Boylann, Encyclopedia of pharmaceutical technology, Marcel Dekker, 1990, Vol. 2, pp 269 ff.
d. After encapsulation, the traces of the lubricant medium-chain triglycerides are removed from the capsule surface, using ethanol denatured with acetone, containing small quantities of Phosal® 53 MCT, used here as anti-sticking agent.
e. The initial drying is carried out using a rotary dryer. For the final drying step, capsules are placed on trays. Drying is performed at 15 - 26°C and low relative humidity.
f. After 100% visual inspection of the capsules for separation of deformed or leaking capsules, the capsules are size sorted and further washed using ethanol denatured with acetone.
g. Finally, the capsules are imprinted, using an Offset printing technology or an Ink-jet printing technology. Alternatively, the capsule imprint can be made using the Ribbon printing technology, a technology in which the gelatin bands are imprinted prior to the encapsulation step c.

Compound B1 (pemetrexed) may be administered according to known clinical practice. For example in NSCLC, the recommended dose of pemetrexed is 500mg/m² given by 10 minute intravenous infusion, administered on the first day of each 21-day cycle.

The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of y-rays, X-rays, and/or the directed delivery of radiation from radioisotopes. Other forms of DNA damaging factors are also included in the present invention such as microwaves and UV- irradiation. For example X-rays may be dosed in daily doses of 1.8-2.0 Gy, 5 days a week for 5-6 weeks. Normally a total fractionated dose will lie in the range 45-60 Gy. Single larger doses, for example 5-10 Gy may be administered as part of a course of radiotherapy. Single doses may be administered intraoperatively. Hyperfractionated radiotherapy may be used whereby small doses of X-rays are administered regularly over a period of time, for example 0.1 Gy per hour over a number of days. Dosage ranges for radioisotopes vary widely, and depend on the half-life of the isotope, the strength and type of radiation emitted, and on the uptake by cells.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

## Claims

1. Pharmaceutical combination comprising the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof and the compound *N*-[4-[2-(2-amino-4,7-dihydro-4-oxo-1*H*-pyrrolo[2,3-*d*]pyrimidin-5-yl)ethyl]benzoyl]- L-Glutamic acid or a pharmaceutically acceptable salt thereof for use in a method of treatment of malignant pleural or peritoneal mesothelioma.

2. Pharmaceutical combination for use according to claim 1, wherein the pharmaceutical combination is for use in a method of treatment of malignant pleural mesothelioma.

3. Pharmaceutical combination for use according to claim 1 or 2, in which the pharmaceutically acceptable salt of the compound 3-*Z*-[1-(4-(*N*-((4-methyl-piperazin-1-yl)-methylcarbonyl)-*N*-methyl-amino)-anilino)-1-phenylmethylene]-6-methoxycarbonyl-2-indolinone is its monoethanesulphonate salt form.

4. Pharmaceutical combination for use according to claim 1 or 2, in which the pharmaceutically acceptable salt of the compound *N*-[4-[2-(2-amino-4,7-dihydro-4-oxo-1*H*-pyrrolo[2,3-*d*]pyrimidin-5-yl)ethyl]benzoyl]-L-Glutamic acid is its disodium salt form.

5. Pharmaceutical combination for use according to claim 1 or 2, comprising the monoethanesulphonate salt form of the compound 3-Z-[1-(*4*-(*N*-((*4-*methyl-piperazin-1-yl)-methylcarbonyl)-*N*-methyl-amino)-anilino)-1-phenylmethylene]-6-methoxycarbonyl-2-indolinone and the disodium salt form of the compound *N*-[4-[2-(2-amino-4,7-dihydro-4-oxo-1*H*-pyrrolo[2,3-*d*]pyrimidin-5-yl)ethyl]benzoyl]-L-Glutamic acid.

6. The pharmaceutical combination for use according to any one of claims 1 to 5, which is in the form of a combined preparation for simultaneous, separate or sequential use.

7. The pharmaceutical combination for use according to any one of claims 1 to 5, which is further adapted for a co-treatment with radiotherapy.

8. The compound 3-*Z*-[1-(4-(*N*-((4-methyl-piperazin-1-yl)-methylcarbonyl)-*N-*methyl-amino)-anilino)-1-phenyl-methylene]-6-methoxycarbonyl-2-indolinone or a pharmaceutically acceptable salt thereof for use in a method of treatment of malignant pleural or peritoneal mesothelioma comprising administering to a patient in need thereof an effective amount of said compound before, after or simultaneously with an effective amount of the compound *N*-[4-[2-(2-amino-4,7-dihydro-4-oxo-1*H*-pyrrolo[2,3-*d*]pyrimidin-5-yl)ethyl]benzoyl]-L-Glutamic acid or a pharmaceutically acceptable salt thereof.

9. The compound for use according to claim 8 wherein the compound is for use in a method of treatment of malignant pleural mesothelioma.

10. The compound for use according to claim 8 or 9, wherein the pharmaceutically acceptable salt of the compound 3-*Z*-[1-(4-(*N*-((4-methyl-piperazin-1-yl)-methylcarbonyl)-*N*-methyl-ami-no)-anilino)-1-phenyl-methylene]-6-methoxycarbonyl-2-indolinone is its monoethane-sulfonate salt.

11. The compound for use according to claim 8 or 9, wherein the pharmaceutically acceptable salt of the compound *N*-[4-[2-(2-amino-4,7-dihydro-4-oxo-1*H*-pyrrolo[2,3-*d*]pyrimidin-5-yl)ethyl]benzoyl]-L-Glutamic acid is its disodium salt.

## Patentansprüche

1. Pharmazeutische Kombination, umfassend die Verbindung 3-Z-[1-(4-(N-((4-Methylpiperazin-1-yl)-methylcarbonyl)-N-methyl-amino)-anilino)-1-phenyl-methylen]-6-methoxycarbonyl-2-indolinon oder ein pharmazeutisch akzeptables bzw. annehmbares Salz hiervon und die Verbindung N-[4-[2-(2-Amino-4,7-dihydro-4-oxo-1H-pyrrolo[2,3-d]pyrimidin-5-yl)ethyl]benzoyl]-L-glutaminsäure oder ein pharmazeutisch akzeptables bzw. annehmbares Salz hiervon zur Verwendung in einem Verfahren zur Behandlung von malignem Pleura- oder Peritoneal-Mesotheliom.

2. Pharmazeutische Kombination zur Verwendung nach Anspruch 1, wobei die pharmazeutische Kombination zur Verwendung in einem Verfahren zur Behandlung von malignem Pleura-Mesotheliom ist.

3. Pharmazeutische Kombination zur Verwendung nach Anspruch 1 oder 2, wobei das pharmazeutisch akzeptable bzw. annehmbare Salz der Verbindung 3-Z-[1-(4-(N-((4-Methylpiperazin-1-yl)-methylcarbonyl)-N-methyl-amino)-anilino)-1-phenyl-methylen]-6-methoxycarbonyl-2-indolinon die Monoethansulfonatsalzform darstellt.

4. Pharmazeutische Kombination zur Verwendung nach Anspruch 1 oder 2, wobei das pharmazeutisch akzeptable bzw. annehmbare Salz der Verbindung N-[4-[2-(2-Amino-4,7-dihydro-4-oxo-1H-pyrrolo[2,3-d]pyrimidin-5-yl)ethyl]benzoyl]-L-glutaminsäure die Dinatriumsalzform darstellt.

5. Pharmazeutische Kombination zur Verwendung nach Anspruch 1 oder 2, umfassend die Monoethansulfonatsalzform der Verbindung 3-Z-[1-(4-(N-((4-Methyl-piperazin-1-yl)-methylcarbonyl)-N-methyl-amino)-anilino)-1-phenyl-methylen]-6-methoxycarbonyl-2-indolinon und die Dinatriumsalzform der Verbindung N-[4-[2-(2-Amino-4,7-dihydro-4-oxo-1H-pyrrolo[2,3-d]pyrimidin-5-yl)ethyl]benzoyl]-L-glutaminsäure.

6. Pharmazeutische Kombination zur Verwendung nach irgendeinem der Ansprüche 1 bis 5, die in Form einer kombinierten Zubereitung zur gleichzeitigen, getrennten oder aufeinanderfolgenden Verwendung vorliegt.

7. Pharmazeutische Kombination zur Verwendung nach irgendeinem der Ansprüche 1 bis 5, die weiterhin für die Co-Behandlung mit Strahlentherapie angepasst ist.

8. Die Verbindung 3-Z-[1-(4-(N-((4-Methyl-piperazin-1-yl)-methylcarbonyl)-N-methyl-amino)-anilino)-1-phenyl-methylen]-6-methoxycarbonyl-2-indolinon oder ein pharmazeutisch akzeptables bzw. annehmbares Salz hiervon zur Verwendung in einem Verfahren zur Behandlung von malignem Pleura- oder Peritoneal-Mesotheliom, umfassend das Verabreichen einer wirksamen Menge der Verbindung an einen Patienten mit Bedarf hierfür vor, nach oder gleichzeitig mit einer wirksamen Menge der Verbindung N-[4-[2-(2-Amino-4,7-dihydro-4-oxo-1H-pyrrolo[2,3-d]pyrimidin-5-yl)ethyl]benzoyl]-L-glutaminsäure oder einem pharmazeutisch akzeptablen bzw. annehmbaren Salz hiervon.

9. Verbindung zur Verwendung nach Anspruch 8, wobei die Verbindung zur Verwendung in einem Verfahren zur Behandlung von malignem Pleura-Mesotheliom ist.

10. Verbindung zur Verwendung nach Anspruch 8 oder 9, wobei das pharmazeutisch akzeptable bzw. annehmbare Salz der Verbindung 3-Z-[1-(4-(N-((4-Methyl-piperazin-1-yl)-methylcarbonyl)-N-methyl-amino)-anilino)-1-phenyl-methylen]-6-methoxycarbonyl-2-indolinon das Monoethansulfonatsalz darstellt.

11. Verbindung zur Verwendung nach Anspruch 8 oder 9, wobei das pharmazeutisch akzeptable bzw. annehmbare Salz der Verbindung N-[4-[2-(2-Amino-4,7-dihydro-4-oxo-1H-pyrrolo[2,3-d]pyrimidin-5-yl)ethyl]benzoyl]-L-glutaminsäure das Dinatriumsalz darstellt.

## Revendications

1. Association pharmaceutique comprenant le composé 3-Z-[1-(4(N-((4-méthyl-pipérazin-1-yl)-méthylcarbonyl-N-méthyl-amino)-anilino)-1-phényl-méthylène]-6-méthoxycarbonyl-2-indolinone ou un sel pharmaceutiquement acceptable de celui-ci et le composé d'acide *N*-[4-[2-(2-amino-4,7-dihydro-4-oxo-1H-pyrrolo[2,3-*d*]pyrimidin-5-yl)éthyl]benzoyl]-L-glutamique ou un sel pharmaceutiquement acceptable de celui-ci pour son utilisation dans un procédé de traitement d'un mésothéliome pleural ou péritonéal malin.

2. Association pharmaceutique pour son utilisation selon la revendication 1, dans laquelle la Association pharmaceutique est pour son utilisation dans un procédé de traitement d'un mésothéliome pleural malin.

3. Association pharmaceutique pour son utilisation selon la revendication 1 ou 2, dans laquelle le sel pharmaceutiquement acceptable du composé 3-Z-[1-(4(N-((4-méthyl-pipérazin-1-yl)-méthylcarbonyl-*N*-méthyl-amino)-anilino)-1-phényl-méthylène]-6-méthoxycarbonyl-2-indolinone est sa forme de sel monoéthanesulphonate.

4. Association pharmaceutique pour son utilisation selon la revendication 1 ou 2, dans laquelle le sel pharmaceutiquement acceptable du composé d'acide N-[4-[2-(2-amino-4,7-dihydro-4-oxo-1*H*-pyrrolo[2,3-*d*]pyrimidin-5-yl)éthyl]benzoyl]-L-glutamique est sa forme de sel disodique.

5. Association pharmaceutique pour son utilisation selon la revendication 1 ou 2, comprenant la forme de sel monoéthanesulphonate du composé 3-*Z*-[1-(4(*N*-((4-méthyl-pipérazin-1-yl)-méthylcarbonyl-*N*-méthyl-amino)-anilino)-1-phényl-méthylène]-6-méthoxycarbonyl-2-indolinone et la forme de sel disodique du composé d'acide *N*-[4-[2-(2-amino-4,7-dihydro-4-oxo-1*H-*pyrrolo[2,3-*d*]pyrimidin-5-yl)éthyl]benzoyl]-L-glutamique.

6. Association pharmaceutique pour son utilisation selon l'une quelconque des revendications 1 à 5, qui est sous forme d'une préparation combinée pour une utilisation simultanée, séparée ou séquentielle.

7. Association pharmaceutique pour son utilisation selon l'une quelconque des revendications 1 à 5, qui est en outre adaptée pour un co-traitement avec une radiothérapie.

8. Composé 3-*Z*-[1-(4(*N*-((4-méthyl-pipérazin-1-yl)-méthylcarbonyl-*N*-méthyl-amino)-anilino)-1-phényl-méthylène]-6-méthoxycarbonyl-2-indolinone ou un sel pharmaceutiquement acceptable de celui-ci pour son utilisation dans un procédé de traitement d'un mésothéliome pleural ou péritonéal malin comprenant l'administration à un patient en ayant besoin d'une quantité efficace dudit composé avant, après ou simultanément avec une quantité efficace du composé d'acide *N*-[4-[2-(2-amino-4,7-dihydro-4-oxo-1*H-*pyrrolo[2,3-*d*]pyrimidin-5-yl)éthyl]benzoyl]-L-glutamique ou un sel pharmaceutiquement acceptable de celui-ci.

9. Composé pour son utilisation selon la revendication 8, dans lequel le composé est pour son utilisation dans un procédé de traitement d'un mésothéliome pleural malin.

10. Composé pour son utilisation selon la revendication 8 ou 9, dans lequel le sel pharmaceutiquement acceptable du composé 3-Z-[1-(4(N-((4-méthyl-pipérazin-1-yl)-méthylcarbonyl-N-méthyl-amino)-anilino)-1-phényl-méthylène]-6-méthoxycarbonyl-2-indolinone est son sel monoéthane-sulphonate.

11. Composé pour son utilisation selon la revendication 8 ou 9, dans lequel le sel pharmaceutiquement acceptable du composé d'acide N-[4-[2-(2-amino-4,7-dihydro-4-oxo-1*H*-pyrrolo[2,3-*d*]pyrimidin-5-yl)éthyl]benzoyl]-L-glutamique est son sel disodique.
